Europäisches Patentamt

European Patent Office

Office européen des brevets

(19)

(11) Veröffentlichungsnummer: **0 036 546**
**B1**

(12) **EUROPÄISCHE PATENTSCHRIFT**

(45) Veröffentlichungstag der Patentschrift:
**27.07.83**

(21) Anmeldenummer: **81101704.5**

(22) Anmeldetag: **09.03.81**

(51) Int. Cl.³: **A 61 M 1/00**

(54) **Saugflasche zum Absaugen von Sekreten aus Wundhöhlen.**

(30) Priorität: **22.03.80 DE 3011163**

(43) Veröffentlichungstag der Anmeldung:
**30.09.81 Patentblatt 81/39**

(45) Bekanntmachung des Hinweises auf die Patenterteilung:
**27.07.83 Patentblatt 83/30**

(84) Benannte Vertragsstaaten:
**BE DE FR GB NL**

(56) Entgegenhaltungen:
**DE-U-7 336 232**
**FR-A-2 447 202**

(73) Patentinhaber: **Sterimed Gesellschaft für medizinischen Bedarf mbH, Fasanerieweg 15, D-6600 Saarbrücken (DE)**

(72) Erfinder: **Härle, Anton, Dr., Schelmenstiege 8, D-4400 Münster-Roxel (DE)**

Saugflasche zum Absaugen von Sekreten aus Wundhöhlen

Die Erfindung bezieht sich auf eine Saugflasche zum Absaugen von Sekreten aus Wundhöhlen mit einem mit dem Flascheninneren in Verbindung stehenden Druckerzeuger zum Überprüfen des Unterdruckes und einem Anschlußstutzen für einen Saugschlauch.

In dem DE-U-7 336 232 wird eine Saugflasche aus Kunststoff beschrieben, die zum Absaugen von Sekreten aus Wundhöhlen dient, wobei die Saugflasche ein Anschlußteil zum Anbringen des eigentlichen Saugschlauches aufweist und mit einem Druckanzeiger kombiniert ist. Der Druckanzeiger ist bei der bekannten Vorrichtung als Faltenbalg ausgebildet und soll durch seine entsprechende Ausdehnung dem Arzt oder Pflegepersonal anzeigen, welcher Unterdruck in der Saugflasche vorhanden ist. In der Praxis hat sich herausgestellt, daß der Einsatz von Faltenbälgen als Druckanzeigevorrichtung ungenau ist, wobei insbesondere bei längerem Lagern eine Materialermüdung eintritt, die zu Ungenauigkeiten der Anzeige und damit zu Fehlschlüssen des Arztes oder Pflegepersonals führt.

In dem DE-U-7 902 325 wird ebenfalls eine Saugflasche für die Wunddrainage beschrieben, wobei die Saugflasche mit einem als Faltenbalg ausgebildeten Druckanzeiger ausgerüstet ist, aber zusätzlich einen Skalenträger aufweist, der mit der Druckanzeigevorrichtung kombiniert ist. wodurch die Druckanzeige sicherer abschätzbar gemacht werden soll.

Der Erfindung liegt die Aufgabe zugrunde, die Druckanzeige bei derartigen vorstehend beschriebenen Saugflaschen so zu verbessern, daß die Druckanzeige genau ist und somit ein sicheres Anzeigen für die in der Saugflasche herrschende Druckhöhe gibt.

Diese der Erfindung zugrunde liegende Aufgabe wird gelöst durch eine aus federelastischem Werkstoff gefertigte Rückstellfeder für den Druckanzeiger, die sich außerhalb eines die Drucköffnung umschließenden an seinem einen Ende an der Saugflasche abgedichtet festgelegten, an seinem anderen Ende verschlossenen Dichtschlauches zwischen der Oberseite der Saugflasche und einem am Dichtschlauch festgelegten Widerlager abstützt. Vorzugsweise besteht die aus federelastischem Werkstoff gefertigte Rückstellfeder aus Stahl, da derartige Stahlfedern eine ausgezeichnete Federkonstanz aufweisen und gleichzeitig kostengünstig beziehbar sind.

Der Einsatz metallischer Bauteile in Verbindung mit Saugflaschen war bisher von der Fachwelt abgelehnt worden, da bei derartigen metallischen Bauteilen sichergestellt werden mußte, daß diese nicht in die Entsorgungsanlage, die üblicherweise aus Verbrennungsöfen besteht, gelangen dürfen. Der Einbau einer metallischen Rückstellfeder, beispielsweise zum Ersatz des Faltenbalges, bereitete daher Schwierigkeiten, da weiterhin sichergestellt werden muß, daß beispielsweise bei Abnahme der metallischen Feder vor Verwerfen der gefüllten Saugflasche sichergestellt sein muß, daß die Saugflaschenöffnungen verschlossen sind, so daß ein Austreten von keimhaltigem Wundsekret ausgeschlossen war. Der Erfinder mußte also die Aufgabe lösen, einerseits eine Rückstelleinrichtung zu schaffen, die eine sehr hohe Federkonstanz aufweist und damit die Anzeigervorrichtung sicher arbeitend macht, andererseits mußte dafür Sorge getragen werden, daß diese Rückstellfeder abgenommen werden kann und daß trotzdem die Saugflasche hermetisch verschlossen bleibt.

Durch die in den Ansprüchen weiterhin genannten Merkmalen werden alle diese Probleme in besonders vorteilhafter Weise gelöst, und zwar einerseits vorteilhaft für das die Saugflasche handhabende Pflegepersonal andererseits vorteilhaft für die Saugflasche herstellende Produktionsstätte, so daß es möglich ist, eine kostengünstig erstellte Saugflasche dem Pflegepersonal zur Verfügung zu stellen.

Ausgehend von der grundsätzlichen Überlegung gemäß der vorliegenden Erfindung, daß die Rückstellfeder außerhalb eines die Drucköffnung der Saugflasche verschließenden Schlauches angeordnet sein muß, wird weiterhin vorgeschlagen, daß das die Rückstellfeder gegenüber der Flasche abstützende Element, das sogenannte Widerlager, auswechselbar ist, so daß damit ein Lösen dieses Widerlagers vom Dichtschlauch gewährleistet wird. Hierdurch wird erreicht, daß ein Auswechseln der Feder beispielsweise zur Erzielung unterschiedlicher Federkonstanten möglich ist, so daß es möglich wird, Saugflaschen mit unterschiedlichen Drükken zu liefern, wobei die Anzeigevorrichtung der jeweiligen Ausgangsdruckhöhe in der Flasche angepaßt werden kann. Hierbei kann selbstverständlich auch so vorgegangen werden, daß anstelle einer einzigen Rückstellfeder zwei oder mehrere Rückstellfedern kombiniert werden, so daß unterschiedlich federnde Bereiche, z. B. zuerst ein Feinmeßbereich und dann ein groberer Meßbereich in der Anzeigevorrichtung geschaffen werden.

Ausführungsbeispiele der Erfindung werden nachfolgend anhand der Zeichnung erläutert. Die Zeichnung zeigt dabei in

Fig. 1 schaubildlich die Ansicht auf ein Oberteil einer Saugflasche gemäß der Erfindung, in

Fig. 2 eine Ansicht gemäß Fig. 1, wobei der Druckanzeiger teilweise geschnitten ist, in

Fig. 3 in einer auseinandergezogenen Darstellung die Bauteile des eigentlichen Druckanzeigers, in

Fig. 4 in einer Schnittdarstellung eine erste Ausführungsform des Widerlagers und in

Fig. 5 in einer Schnittdarstellung eine zweite Ausführungsform des Widerlagers.

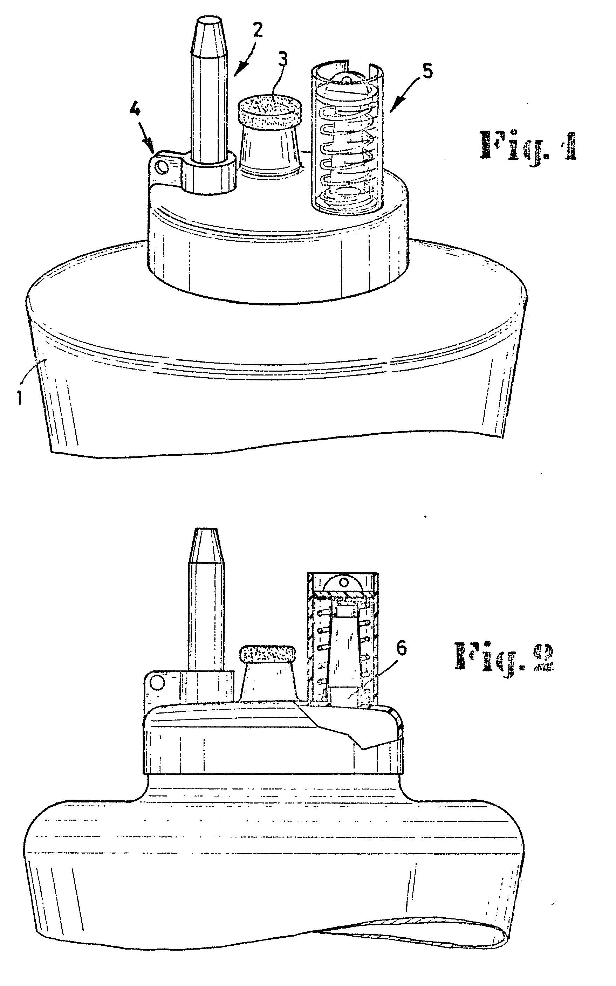

In Fig. 1 ist mit 1 die eigentliche Saugflasche bezeichnet, die an ihrer Oberseite einen Anschlußstutzen 2 für die Saugleitung aufweist. Bei 3 ist eine Durchsteckkappe dargestellt, die es ermöglicht, durch Punktion mit einer Untersuchungskanüle das Innere der Saugflasche bei Aufrechterhaltung des Unterdrucks und der sterilen Verhältnisse zu untersuchen. Die der Halterung der Saugflasche dienende Aufhängeöse ist bei 4 dargestellt. Derartige Saugflaschen gehören zum Stand der Technik.

Mit der Saugflasche ist an der Oberseite der Saugflasche auch ein Druckanzeiger 5 kombiniert, der aber selbstverständlich auch an einer anderen Stelle der Saugflasche angeordnet sein kann.

Der Druckanzeiger 5 besteht im wesentlichen aus einer in der Saugflasche selbst vorgesehenen Drucköffnung 6, einem Dichtschlauch 7, einem Widerlager 8 sowie einer Rückstellfeder 9 und — bei dem dargestellten Ausführungsbeispiel nur — einem Skalenträger 10, wobei diese Bauteile deutlicher aus der Darstellung in Fig. 3 erkennbar sind.

Die Drucköffnung 6 in der Saugflasche ist bei dem dargestellten Ausführungsbeispiel stutzenartig ausgebildet, d. h. es ist ein Ringstutzen 11 an der Oberseite der eigentlichen Saugflasche 1 vorgesehen. Über diesen Ringstutzen 11 kann ein L-förmiger Befestigungsring 12 gesetzt werden, der mit seinem horizontalen Ringbereich 14 an der Oberseite der Saugflasche 1 verklebt werden kann, während der vertikale Ringbereich 15 an seiner Außenseite den Dichtschlauch 7 abgedichtet trägt, während die Innenseite dieses Ringbereiches 15 abgedichtet am Stutzen 11 festgelegt wird.

Der Dichtschlauch 7 kann aus einfacher Folie bestehen, die mit Stabilisierungsringen o. dgl. gegen Kollabieren gesichert ist und weist eine Länge auf, die größer als der Federweg ist und hat lediglich die Aufgabe, die Drucköffnung 6 und damit den im Flascheninneren herrschenden Druck gegenüber dem Umgebungsdruck abzudichten. Hierzu erfolgt einerseits eine druckdichte Festlegung an dem Ringbereich 15, andererseits eine dichte Festlegung des oberen Endes des Dichtschlauches 7 an einem etwas konisch gestalteten Verschlußstopfenteil 16 des Widerlagers 8. An den Verschlußstopfen 16 des Widerlagers 8 schließt sich nach oben hin der eigentliche Abstützungsbereich 17 des Widerlagers 8 an, wobei an diesem Abstützungsbereich 17 die Rückstellfeder 9 anliegt, die sich unten auf der Oberseite des horizontalen Ringbereiches 14 abstützt.

Die Rückstellfeder hält damit normalerweise, d. h., wenn kein Unterdruck in der Flasche 1 vorhanden ist, das Widerlager 8 in seiner oberen Stellung.

Herrscht in der Flasche 1 ein Unterdruck, so ist dieser bestrebt, das Widerlager 8 nach unten zur Drucköffnung 6 hinzuziehen, und zwar gegen die Wirkung der Rückstellfeder 9.

Der Skalenträger 10 weist an seiner Außenseite eine Meßskaleneinteilung auf, die der gewünschten Ablesegenauigkeit entsprechend gewählt wird. Zusätzlich weist aber der Skalenträger gemäß dem in Fig. 3 dargestellten Ausführungsbeispiel Rastnuten 18 und 21 auf, die mit Rastnocken 19 und 20 des Widerlagers 8 so zusammenwirken, daß dann, wenn die Rastnocken 19 und 20 in die Rastnuten 18 und 21 durch Drehen des Widerlagers 8 eingeführt sind, eine Festlegung des Widerlagers 8 in der obersten Stellung erfolgt. Eine solche Anordnung hat den Vorteil, daß bei langer Lagerung der Saugflasche und dem darin herrschen Unterdruck eine Belastung der eigentlichen Rückstellfeder nicht erfolgt, sondern die Rückstellfeder wird erst belastet, wenn das Widerlager 8 durch Drehen so bewegt wird, daß die Rastnocken 19 und 20 aus den zugeordneten Rastnuten 18 und 21 herausgedreht werden, so daß sich dann die Rastnocken 19 und 20 in den Führungsrillen 22 und 23 nach unten bewegen können.

Bei der Darstellung in Fig. 4 ist das Widerlager 8 so dargestellt, daß sich an den Abstützungsbereichen 17 der Verschlußstopfen 16 über einen dünnen Zapfen 24 anschließt. Es ist offensichtlich, daß dann, wenn die Außenseite des Verschlußstopfens 16 festgehalten wird, der eigentliche Abstützungsbereich 17 durch Drehen (unter Zuhilfenahme des Griffteiles 25) und Abscheren des Zapfens 24 abgedreht werden kann. Hierdurch ist ein Lösen des den Verschlußstopfen 16 übergreifenden Abstützungsbereiches 17 vom Widerlager möglich möglich, so daß dann das Entnehmen der Rückstellfeder möglich gemacht wird. Der verbleibende Verschlußstopfen 16 kann dann bei gleichzeitigem Zusammenpressen des Dichtschlauches 7 in die Öffnung des Bauteiles 12 eingepreßt werden und verschließt damit den Innenraum der Dichtflasche 1, so daß ein Ausfließen von Wundsekret nicht möglich ist und außerdem eine Verbindung zwischen Flascheninnerem und Atmosphäre nicht erfolgen konnte, so daß ein sicheres Abdichten einerseits des Flascheninneren gegenüber der Außenatmosphäre gewährleistet wird, andererseits aber auch die Möglichkeit gegeben wird, daß eine spätere Untersuchung des Flascheninhaltes auf Keimzahl und -art nicht durch Kontakt des Flascheninhaltes mit der Außenatmosphäre unmöglich gemacht wird.

Der Skalenträger 10 kann, wie dies durch den Ringflansch 26 dargestellt ist, auf der Oberseite der Saugflasche fest und/oder lösbar angeordnet werden.

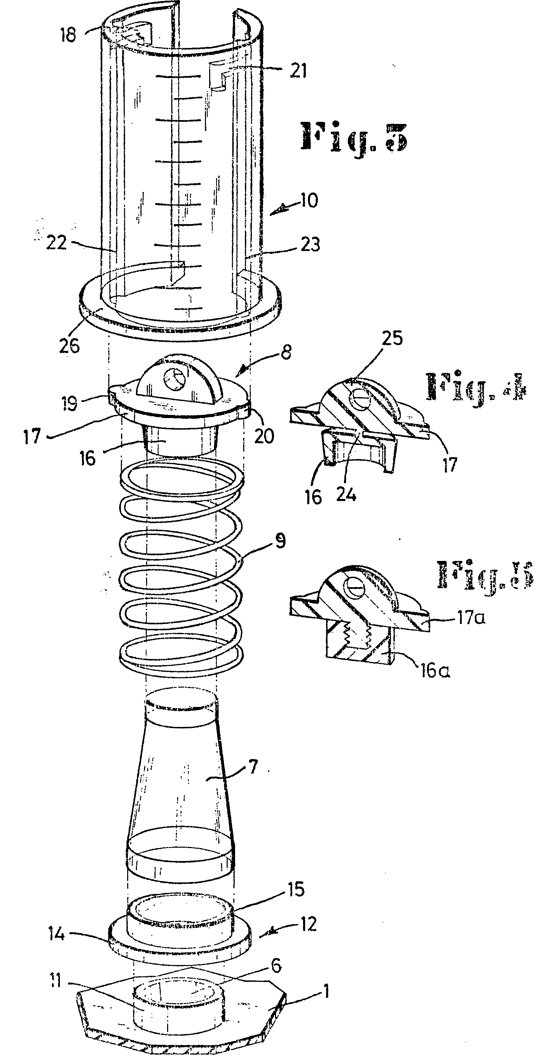

Bei der Darstellung in Fig. 5 wird verdeutlicht, daß die nichtwiederholbare Lösung zwischen Verschlußstopfen 16 und Abstützungsbereich 17 des Widerlagers 8 auch so gestaltet werden kann, daß der Abstützungsbereich 17a lösbar und wiederholbar am Stopfen 16a festgelegt wird. Diese wiederholbare Lösbarkeit kann durch einen Schraubverschluß, Bajonettverschluß, Steckverschluß o. dgl. erreicht werden. Diese Anordnung bietet die Möglichkeit, Federn am Einsatzort einzubauen oder auszuwechseln, so daß damit die Möglichkeit gegeben wird, Saugflaschen ohne Feder an die Verbraucherkrankenhäuser zu liefern, wobei die einzelnen Krankenhäuser einen Federvorrat haben, der nunmehr eingesetzt wird, so daß dadurch die Transportkosten verringert werden können.

Weiterhin schafft die in Fig. 5 dargestellte Lösungsmöglichkeit den Vorteil, daß Ärzte sich ihren Wünschen entsprechende Federkonstanten einbauen können.

## Patentansprüche

1. Saugflasche zum Absaugen von Sekreten aus Wundhöhlen mit einem mit dem Flascheninneren in Verbindung stehenden Druckanzeiger (5) zum Überprüfen des Unterdruckes und einem Anschlußstutzen (2) für einen Saugschlauch, gekennzeichnet durch eine aus federelastischem Werkstoff gefertigte Rückstellfeder (9) für den Druckanzeiger (5), die sich außerhalb eines die Drucköffnung (6) verschließenden an seinem einen Ende an der Saugflasche (1) abgedichtet festgelegten, an seinem anderen Ende verschlossenen Dichtschlauches (7) zwischen der Oberseite der Saugflasche (1) und einem am Dichtschlauch (7) festgelegten Widerlager (8) abstützt.

2. Saugflasche nach Anspruch 1, dadurch gekennzeichnet, daß das Widerlager (8) als mit einem an sich bekannten Skalenträger (10) zusammenwirkende Anzeigevorrichtung ausgebildet ist.

3. Saugflasche nach Anspruch 1 und 2, dadurch gekennzeichnet, daß das Widerlager (8) zwei voneinander trennbare Bereiche aufweist, von denen der eine — den anderen Bereich übergreifend — der Rückstellfeder als Abstützung (17) und der andere Bereich (16) zur Festlegung des Dichtschlauches dient.

4. Saugflasche nach Anspruch 3, dadurch gekennzeichnet, daß der der Festlegung des Dichtschlauches (7) dienende Bereich (16) gleichzeitig als Verschlußstopfen (16) für die Drucköffnung (6) dimensioniert ist.

5. Saugflasche nach Anspruch 3, dadurch gekennzeichnet, daß die Verbindung der beiden Bereiche wiederholbar lösbar gestaltet ist.

6. Saugflasche wenigstens nach Anspruch 1 und 2, dadurch gekennzeichnet, daß der Skalenträger (10) an seinem oberen Ende mit einem Rastverschluß (18, 21) für das Widerlager (8) ausgerüstet ist.

7. Saugflasche nach einem oder mehreren der vorhergehenden Ansprüche, dadurch gekennzeichnet, daß mehrere unterschiedliche Federkonstanten aufweisende und in den Unterdruckanzeiger (5) einsetzbare Rückstellfedern (9) vorgesehen sind.

8. Saugflasche wenigstens nach Anspruch 1 und 2, dadurch gekennzeichnet, daß mehrere Skalenträger mit unterschiedlich gestuften Skalen vorgesehen sind.

## Claims

1. Aspiration bottle for withdrawing, by suction, secretions from wound cativies, with a pressure indicator (5), connected to the inside of the bottle, for monitoring the reduced pressure, and a connecting piece (2) for an aspiration tube, characterised by a restoring spring (9) for the pressure indicator (5), which spring is made of resilient material and is supported, outside the sealing tube (7) which closes the pressure opening (6) and at one end is sealed to the aspiration bottle (1) and is closed at the other end, between the top of the aspiration bottle (1) and an abutment (8) fixed to the sealing tube (7).

2. Aspiration bottle according to Claim 1, characterised in that the abutment (8) is designed as a display device which acts together with a scale carrier (10) which is known per se.

3. Aspiration bottle according to Claim 1 and 2, characterised in that the abutment (8) has two areas, separated from one another, one of which — overlapping the other area — serves as a support (17) for the restoring spring and the other area (16) of which serves to fix the sealing tube.

4. Aspiration bottle according to Claim 3, characterised in that the area (16) serving to fix the sealing tubes (7) at the same time has the dimensions of a sealing stopper for the pressure opening (6).

5. Aspiration bottle according to Claim 3, characterised in that the connection between the two areas is designed such that it can be repeatedly broken.

6. Aspiration bottle at least according to Claim 1 and 2, characterised in that the upper end of the scale carrier (10) is provided with a catch-seal (18, 21) for the abutment (8).

7. Aspiration bottle according to one or more of the preceding claims, characterised in that several restoring spring (9) which have different spring constants and can be used in the pressure indicator (5) are provided.

8. Aspiration bottle at least according to Claims 1 and 2, characterised in that several scale carriers with scales having different graduations are provided.

## Revendications

1. Flacon aspirant pour l'enlèvement de sécrétions des plaies comprenant un indicateur de pression debout (5) en liaison avec l'intérieur du flacon pour examiner la dépression et un embout de raccordement (2) pour un tuyau d'aspiration, caractérisé par un ressort de rappel (9) en matière élastique pour l'indicateur de pression (5), lequel ressort s'appuie à l'extérieur d'un tube étanche (7) fermant l'ouverture de pression (6), ayant une extrémité fixée hermétiquement au flacon aspirant (1) et étant fermée à son autre extrémité, et s'appuie entre la face supérieure du flacon aspirant (1) et une butée (8) fixée au tuyau étanche (7).

2. Flacon aspirant selon la revendication 1, caractérisé en ce que la butée (8) joue le rôle de dispositif indicateur coopérant avec un support gradué connu (10).

3. Flacon aspirant selon les revendications 1 et 2, caractérisé en ce que la butée (8) comprend deux parties séparables l'une de l'autre dont l'une — surplombant l'autre partie — sert d'appui (17) pour le ressort de rappel et dont l'autre (16) sert à la fixation du tuyau étanche.

4. Flacon aspirant selon la revendication 3, caractérisé en ce que la partie (16) servant à la fixation du tuyau étanche (7) est dimensionnée en même temps pour former un bouchon de fermeture (16) de l'ouverture de pression (6).

5. Flacon aspirant selon la revendication 3, caractérisé en ce que la liaison des deux parties est prévue amovible et reproductible.

6. Flacon aspirant selon au moins les revendications 1 et 2, caractérisé en ce que le support gradué (10) est muni à son extrémité supérieure d'un verrouillage d'arrêt (18, 21) pour la butée (8).

7. Flacon aspirant selon l'une ou plusieurs des revendications précédentes, caractérisé en ce que sont prévus plusieurs ressorts de rappel (9) présentant des constantes d'élasticité différentes et insérables dans l'indicateur de pression (5).

8. Flacon aspirant selon au moins les revendications 1 et 2, caractérisé en ce que sont prévus plusieurs supports gradués ayant des échelles de graduation différentes.

Fig. 1

Fig. 2

**Fig. 3**

**Fig. 4**

**Fig. 5**